# EUROPEAN PATENT APPLICATION

(11) **EP 0 994 094 A1**
(43) Date of publication of application: **19.04.2000**
(21) Application number: 99308081.1
(22) Date of filing: 13.10.1999
(51) Int. Cl.: C07C 51/363, C07C 51/43, C07C 59/21

(54) **Process for manufacturing 3-chloropyruvic acid**

(30) Priority: 16.10.1998 JP 29530198; 22.02.1999 JP 4288899
(71) Applicant: Nippon Sanso Corporation, Tokyo (JP)
(72) Inventor: Abe, Toshifumi, c/o Nippon Sanso Corp., Minato-ku, Tokyo (JP)
(74) Representative: Skailes, Humphrey John

(57) **Abstract**

A process for synthesizing 3-chloropyruvic acid that is able to efficiently synthesize 3-chloropyruvic acid by inhibiting the formation of by-products in the form of the dichloride and trichloride. After mixing pyruvic acid and sulfuryl chloride while cooling to a temperature lower than their reaction temperature, and preferably to 0 to 10°C, the reaction temperature is raised and reaction of the components is carried out to obtain 3-chloropyruvic acid. This 3-chloropyruvic acid is then purified by temperature-controlled fractional sublimation.

## Description

### TECHNICAL FIELD

The present invention relates to a process for synthesizing 3-chloropyruvic acid, more particularly to a process for manufacturing 3-chloropyruvic acid by reacting pyruvic acid with sulfuryl chloride.

### BACKGROUND ART

3-Chloropyruvic acid (CH₂ClCOCOOH) is generally synthesized through a reaction between pyruvic acid and sulfuryl which are starting materials. For example, a process for obtaining a viscous pale yellow liquid product is described in Cragoe, Edward J. Jr.; Robb, Charles M., ORGANIC SYNTHESES, P. 54, VOL 40 (1960), in which 394 g (2.92 mol) of sulfuryl is added with stirring to 249 g (2.83 mol) of pyruvic acid, maintained at a temperature of 25 to 30°C in a water bath, over 2 hours, and the resulting mixture is reacted at room temperature with stirring for 60 hours.

However, according to the above process of mixing the starting materials at ambient temperature, large amounts of dichloride (CHCl₂COCOOH) and trichloride (CCl₃COCOOH) are formed as by-products. It is difficult to separate and remove these large amounts of by-products from 3-chloropyruvic acid, and if such a 3-chloropyruvic acid product is used as a raw material for producing amino acids and the like, it exerts an inhibitory effect upon the reaction to be causative of formation of by-products. Under such circumstances, while it is practiced to purify 3-chloropyruvic acid product by removing the unreacted components and by-products, the purification treatment has conventionally been carried out by evacuation at room temperature to remove volatile components only. However, this purification method involves a disadvantage that it can remove only volatile components but cannot remove the residual (unreacted) pyruvic acid and dichloride or trichloride as impurities.

### SUMMARY OF THE INVENTION

A first objective of the present invention is to provide a process capable of synthesizing efficiently 3-chloropyruvic acid, while formation of by-product dichloride or trichloride is inhibited.

A second objective of the present invention is to provide a process for purifying 3-chloropyruvic acid which can remove efficiently residual pyruvic acid and by-product dichloride or trichloride contained as impurities in the synthesized 3-chloropyruvic acid product.

The present invention relates to a process for synthesizing 3-chloropyruvic acid by reacting pyruvic acid with sulfuryl chloride, comprising a step of mixing the above-mentioned pyruvic acid and sulfuryl chloride at a temperature lower than the reaction temperature, and a step of heating the mixture obtained in the preceding step to the reaction temperature to effect reaction between them. Preferably, the temperature lower than the reaction temperature in the above-mentioned mixing step is 0 to 10°C.

According to this process, the formation of by-products, which present a considerable obstacle during synthesis of amino acids and so forth that uses 3-chloropyruvic acid as a raw material, can be inhibited, thereby allowing the obtaining of 3-chloropyruvic acid at high yield.

In addition, the 3-chloropyruvic acid obtained in the above-mentioned reaction step can be purified by temperature-controlled fractional sublimation. It is preferable that the temperature control be performed at a first heating temperature and a second heating temperature above said first heating temperature. The first heating temperature is preferably 40 to 60°C, while the second heating temperature is preferably 65 to 85°C. The component sublimed at the above-mentioned second heating temperature is collected in the form of purified 3-chloropyruvic acid by removing the component that sublimed at the above-mentioned first heating temperature.

According to this method, impurities in the form of unreacted raw materials and by-products can be removed by a simple procedure, and since heating is not performed at a high temperature for an extended period of time,
decomposition and polymerization can be prevented, thereby allowing the obtaining of highly pure 3-chloropyruvic acid at high yield.

### Brief Description of the Drawings

Fig. 1 is a front view showing one example of a different synthesis reaction apparatus used to carry out the process of the present invention at laboratory scale.

Fig. 2 is a cross-sectional view showing one example of a fractional sublimation purification apparatus for carrying out the purification step of the present invention.

Fig. 3 is an NMR chart of 3-chloropyruvic acid synthesized in Experiment 3 of Example 1.

### Best Mode for Carrying Out the Invention

3-chloropyruvic acid (CH₂ClCOCOOH) is synthesized by the following reaction using pyruvic acid (CH₃COCOOH) and sulfuryl chloride (SO₂Cl₂) as raw materials:

CH₃COCOOH + SO₂Cl₂ = CH₂ClCOCOOH + SO₂ + HCl

The above-mentioned pyruvic acid is a liquid at normal temperature having a molecular weight of 88.06, while sulfuryl chloride is a liquid at normal temperature having a molecular weight of 134.97. Reagent guaranteed grade, grade 1 or industrial grade commercial products can be used for these raw materials. In addition, the product 3-chloropyruvic acid is a solid at normal temperature. By-products during synthesis, namely dichloride (CHCl₂COCOOH) and trichloride (CCl₃COCOOH) are also solids at normal temperature.

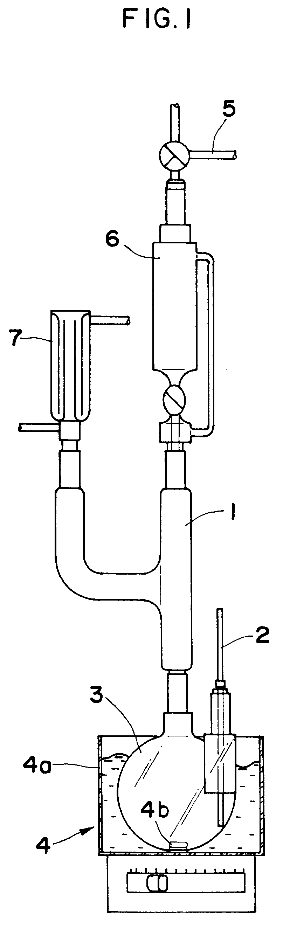

Fig. 1 is a front view showing one example of a synthesis reaction apparatus when carrying out the process of the present invention at laboratory scale. This apparatus consists of a two-neck round-bottom flask 3 equipped with a Claisen adapter 1 and a thermometer 2 placed in a constant-temperature water bath 4 containing a magnetic stirrer, and a dropping funnel 6 equipped with a nitrogen gas feed port 5 and an oil trap 7 attached to the Claisen adapter 1. All connections can be of the ground glass inter-connecting type made of Pyrex (trade name) glass.

Synthesis of 3-chloropyruvic acid is carried out by adding the required amount of pyruvic acid into the two-neck round-bottom flask 3 and the required amount of sulfuryl chloride into the dropping funnel 6, replacing the atmosphere in the apparatus with nitrogen by supplying nitrogen gas through the nitrogen gas feed port 5, and placing ice water in the water bath 4a of the constant-temperature water bath 4 containing a magnetic stirrer or circulating a cooled coolant to cool the two-neck round-bottom flask 3. The reaction starts while stirring the liquid in the flask 3 with a magnetic stirrer 4b.

The amount of sulfuryl chloride dropped in is 1.0 to 1.10, and preferably 1.02 to 1.05, relative to a stoichiometric ratio of 1 of pyruvic acid. If this ratio is less than 1.0, a large amount of raw material pyruvic acid ends up remaining unreacted, while if the ratio exceeds 1.1, the formation of dichloride cannot be controlled.

The cooling temperature of the flask 3 via the water bath 4a should be lower than the reaction temperature (15 to 35°C). Although any such temperature is satisfactory provided it does not have a detrimental effect on the stirring and mixing of both components, it is usually 0 to 10°C and preferably 3 to 7°C. If this cooling temperature is too high, there is increased susceptibility to formation of by-products in the form of dichloride and trichloride. If the cooling temperature exceeds 10°C, for example, by-products are formed at the rate of about 15%, while they are only formed at about 9% at a cooling temperature of 7 to 10°C. On the other hand, if the cooling temperature is less than 3°C, the reaction rate decreases and reaction time is extended. If the cooling temperature is below 3°C, for example, the reaction rate decreases by about 20% as compared with a cooling temperature of 5 to 7°C, and decreases by about 10% even if the cooling temperature is below 5°C. Moreover, the cost required for cooling increases when an even lower cooling temperature is used.

In addition, the suitable dropping rate of sulfuryl chloride is about 0.2 to 0.8 g/minute, and preferably within the range of 0.4 to 0.6 g/minute. If the dropping rate is less than 0.2 g/minute, an excessive amount of time is required to drop in the sulfuryl chloride, thereby increasing the risk of contamination by impurities such as moisture from outside the system. Conversely, if the dropping rate exceeds 0.8 g/minute, there is greater likelihood of formation of dichloride.

After dropping in the entire amount of sulfuryl chloride, stirring is continued for an additional 0.5 to 1.5 hours, and preferably about 1 hour, while maintaining cooling to sufficiently mix the pyruvic acid and sulfuryl chloride.

Next, the reaction according to the above-mentioned reaction formula proceeds by heating the mixture in the flask 3 to the reaction temperature following completion of cooling of the flask 3. As a result, 3-chloropyruvic acid can be synthesized.

The reaction temperature in this reaction step is within the range of 15 to 35°C, and preferably within the range of 20 to 30°C. If the reaction temperature exceeds 35°C, polymerization and other side reactions occur, while if the reaction temperature is below 15°C, the reaction becomes extremely slow. Raising the temperature from the cooling temperature to the reaction temperature can be performed by using a suitable heating means such as a heater, circulating water at a suitable temperature in water bath 4a, or opening up the system to the atmosphere. While the reaction time varies depending on the reaction temperature, it is normally 24 to 120 hours, and preferably 48 to 72 hours. If the reaction time is less than 24 hours, the reaction is not completed, while if the reaction time exceeds 120 hours, polymerization and other side reactions occur.

When synthesizing 3-chloropyruvic acid using this type of procedure, by dropping in sulfuryl chloride into pyruvic acid cooled to a temperature lower than the reaction temperature as described above when mixing, the reaction of both raw materials during mixing can be inhibited and side reactions can be suppressed, thereby virtually eliminating the formation of by-products. By starting the reaction after first raising the temperature following sufficient mixing, both raw materials can be reacted uniformly, thereby also inhibiting side reactions.

The amount of synthesized 3-chloropyruvic acid and the amounts of residual pyruvic acid and by-product dichloropyruvic acid can be quantified from the integrated values of each peak of 1H-NMR (nuclear magnetic resonance). Namely, the amount of 3-chloropyruvic acid can be determined by using the peak having a chemical shift of 4.78 to 4.91 ppm, the amount of residual pyruvic acid can be determined by using the peak having a chemical shift of 2.27 to 2.37 ppm, and the amount of dichloropyruvic acid can be determined by using the peak having a chemical shift of 6.00 to 6.22 ppm in 1H-NMR. In addition, the amount of trichloropyruvic acid can be determined by 13C-NMR.

The above-mentioned 3-chloropyruvic acid obtained following completion of the reaction can be efficiently purified by applying the fractional sublimation method explained below.

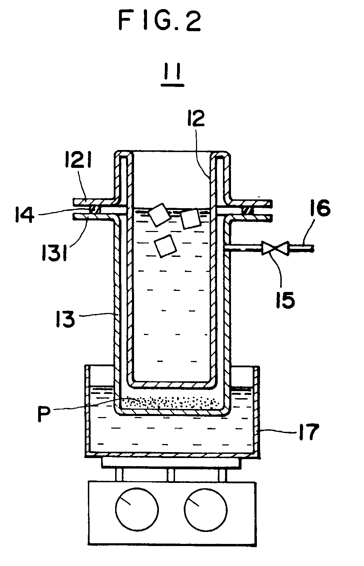

Fig. 2 is a cross-sectional view showing one example of a fractional sublimation purification apparatus used in this method. This fractional sublimation purification apparatus 11 is composed of an internal container 12 and an external container 13 assembled in a sealed state while allowing disassembly with an O-ring 14 juxtaposed between upper peripheral flanges 121 and 131. The internal container 12 fulfills the role of a low-temperature bath in which a cooling medium such as ice water is placed inside. Both internal container 12 and external container 13 may be made of metal or glass, and both may be formed from the same material or different materials. The external container 13 is provided with a vacuum exhaust tube 16 equipped with a valve 15 for drawing a vacuum between the two containers or returning the two containers to atmospheric pressure. In addition, the bottom of the external container 13 is installed in a temperature-controllable constant-temperature bath 17, and the bottom of the external container 13 is formed so that it can be heated to a desired temperature with an oil bath and so forth.

When purifying 3-chloropyruvic acid with the fractional sublimation purification apparatus 11 formed in this manner, 3-chloropyruvic acid P is placed in the bottom of the external container 13, the internal container 12 is attached, and the bottom of the external container 13 is immersed in the constant-temperature bath 17.

It should be noted that the interval between the internal container 12 and the external container 13 is suitably 5 to 150 mm, and preferably 10 to 50 mm. If the interval exceeds 150 mm, cooling efficiency becomes poor, while if the interval is less than 5 mm, the target component is contaminated by scattering of impurities.

Next, the following provides a detailed explanation of the preferable purification step of 3-chloropyruvic acid.

Firstly, 3-chloropyruvic acid synthesized in the reaction step is placed in the external container 13, and the internal container 12 is attached by sealing with the O-ring 14. While the amount of 3-chloropyruvic acid varies depending on the size of the apparatus at this time, it is preferable that the evaporation surface area be within the range of 10 to 400 cm², and the thickness of 20 mm or less, and particularly 10 mm or less. If the thickness exceeds 20 mm, a large amount of substances other than the target component gather near the sublimation surface, thereby inhibiting sublimation of the target component. In addition, the amount of target component adhered to the internal container 12 as a result of sublimation should be kept to a thickness of 10 mm or less, and preferably 5 mm or less. If the thickness exceeds 10 mm, the adhered target component ends up peeling off due to the decrease in cooling efficiency.

Next, after drawing a vacuum between both containers by opening the valve 15 and operating a vacuum pump connected to the vacuum exhaust tube 16, the valve 15 is closed to hold the vacuum. In addition, the walls of the internal container 12 are cooled by placing a low-temperature medium such as ice water or cold water inside the internal container 12. This cooling temperature is suitably within the range of -10 to 20°C, and preferably -5 to 10°C. If cooled to a temperature lower than -10°C, the substance adhering to the outside of the internal container 12 as a result of sublimation becomes excessively hard thereby making it difficult to peel off and remove the substance. If the cooling temperature is above 20°C, the adhered substance becomes too soft, thereby running the risk of falling off when subjected to even a gentle impact.

The first sublimation purification step is then performed at the first heating temperature by heating the oil bath and so forth of the constant-temperature bath 17 to heat the bottom of the external container 13. This step is for separating and removing residual pyruvic acid remaining in the 3-chloropyruvic acid, and the heating temperature at this time (first heating temperature) is 40 to 60°C, and preferably 45 to 55°C. If the temperature is less than 40°C, pyruvic acid as an impurity is not sufficiently sublimed, while if the temperature exceeds 60°C, the product 3-chloropyruvic acid ends up subliming with pyruvic acid. In addition, the duration of the first sublimation purification step is suitably 0.6 to 3 hours. If the duration is less than 0.6 hours, pyruvic acid as an impurity is unable to be sufficiently sublimed, while if the duration exceeds 3 hours, there is the risk of commencement of decomposition and polymerization.

Moreover, the sublimation rate should be 1.5 to 7.5 g/h, and preferably 3.0 to 6.0 g/h, within the above-mentioned temperature and time ranges. If the sublimation rate is less than 1.5 g/h, since the heating time ends up being excessively long as a result, there is the risk of commencement of decomposition and polymerization. If the sublimation rate exceeds 7.5 g/h, the excessively rapid sublimation ends up causing sublimation of the product 3-chloropyruvic acid as well.

After performing the first sublimation purification step in this manner, once pyruvic acid as an impurity is sublimed and adheres to the internal container 12, the temperature of the constant-temperature bath 17 is lowered, or the container is removed from the constant-temperature bath 17 and after allowing to cool to room temperature, the vacuum is broken, the internal and external containers are separated, and the sublimation product (pyruvic acid) adhered to the outside of the internal container 12 is removed.

Next, after similarly attaching the internal container 12 and the external container 13 so that they are sealed, drawing a vacuum between both containers and holding that vacuum, in addition to cooling the internal container 12 with ice water and so forth, the outer container 13 is heated by constant-temperature bath 17 to perform the second sublimation purification step. This second sublimation purification step is for subliming the product 3-chloropyruvic acid. The heating temperature at this time (second heating temperature) is suitably 65 to 85°C, and preferably 70 to 80°C. If the second heating temperature is less than 65°C, hardly any of the product 3-chloropyruvic acid is sublimed, while if the temperature exceeds 85°C, decomposition and polymerization may occur.

The sublimation rate is suitably 5 to 25 g/h and preferably 10 to 20 g/h. If the sublimation rate is less than 5 g/h, the heating time becomes excessively long. Thus, there is the risk of commencement of decomposition and polymerization. If the sublimation rate exceeds 25 g/h, the excessively rapid sublimation causes the dichloride as an impurity to sublime with the 3-chloropyruvic acid in some cases. Moreover, the duration of the second sublimation purification step is suitably 0.6 to 3 hours. If the duration is less than 0.6 hours, it is difficult to adequately sublime the 3-chloropyruvic acid, and if the duration exceeds 3 hours, the prolonged duration of heating results in the risk of commencement of decomposition and polymerization. The cooling temperature of the internal container 12 should be slightly higher than the first step at -10 to 20°C, and preferably 0 to 10°C. If the temperature is too low, volatile components formed due to decomposition end up adhering to the internal container 12 with the product 3-chloropyruvic acid, while if the temperature is too high, sublimed 3-chloropyruvic acid does not adhere to the internal container 12.

As a result, 3-chloropyruvic acid adheres to the internal container 12, while the dichloride and trichloride remain in the bottom of external container 13. Thus, following completion of this second sublimation purification step, purified 3-chloropyruvic acid can be obtained by removing the container from the constant-temperature bath 17, disassembling, and separating and recovering the product adhered to the internal container 12. It should be noted that this recovery procedure should be performed in as short a time as possible to prevent reductions in yield due to evaporation and dissipation of the product caused by rises in temperature.

As a result of purifying 3-chloropyruvic acid by a temperature-controlled fractional sublimation method in this manner, residual pyruvic acid and other impurities such as dichloride can be effectively separated and removed, thereby enabling 3-chloropyruvic acid to be obtained at high yield. Incidentally, although 3-chloropyruvic acid can be purified even if the first sublimation purification step is performed at a slightly higher temperature and the second sublimation purification step is performed at a slightly lower temperature, the yield tends to be lower than that obtained with the above procedure.

The amounts of 3-chloropyruvic acid as well as residual pyruvic acid and by-product dichloropyruvic acid that are contained as impurities in 3-chloropyruvic acid can be determined according to the integrated values of each peak of 1H-NMR (nuclear magnetic resonance). More specifically, 3-chloropyruvic acid can be quantified by the peak having a chemical shift of 4.78 to 4.91 ppm, residual pyruvic acid by that having a chemical shift of 2.27 to 2.37 ppm, and dichloropyruvic acid by that having a chemical shift of 6.00 to 6.22 ppm in 1H-NMR. In addition, the amount of trichloropyruvic acid can be determined by 13C-NMR.

### Example 1

8.81 g (0.100 mol, 1 equivalent) of pyruvic acid (Kanto Chemical, guaranteed grade) were placed in a two-neck flask having an internal volume of 50 ml, and 13.93 g (0.103 mol, 1.03 equivalents) of sulfuryl chloride (Kanto Chemical, guaranteed grade) were placed in a dropping funnel also having an internal volume of 50 ml in a synthesis reaction apparatus having the constitution shown in Fig. 1. Nitrogen gas was allowed to flow into the apparatus for about 1 minute to replace the atmosphere in the apparatus with nitrogen.

The cooling temperature was set at five levels consisting of -4.5°C (Experiment 1), 1.0°C (Experiment 2), 5.5°C (Experiment 3), 9.5°C (Experiment 4) and 12.5°C (Experiment 5), and either coolant was circulated through the water bath or ice water was placed in the water bath to maintain each temperature corresponding to each experimental temperature.

The entire amount of sulfuryl chloride was dropped in over the course of approximately 30 minutes while stirring the pyruvic acid with a stirrer. There were no temperature changes caused by the heat of the reaction observed during this time. Next, after stirring was continued for an additional hour under the cooling state, the water bath was removed and the flask was allowed to stand at room temperature (25°C) to carry out the synthesis reaction for 24 hours while stirring. During the reaction, the mixture in the flask gradually increased in viscosity so that after 24 hours, a reaction product was obtained in the form of a white solid.

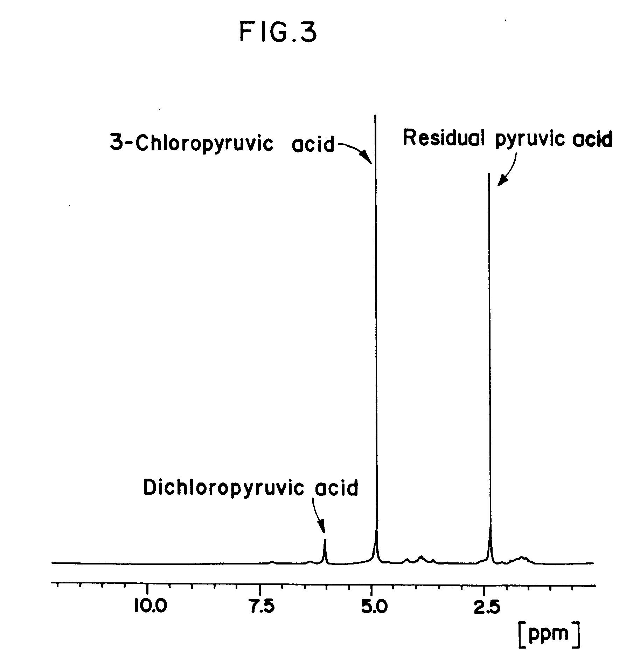

Fig. 3 shows an NMR chart of the reaction product (3-chloropyruvic acid) obtained in Experiment 3. It can be seen from this chart that the composition of the reaction product consists of 82.0 wt% of 3-chloropyruvic acid, 4.7 wt% of dichloropyruvic acid and 13.3 wt% of residual pyruvic acid. The compositions of the reaction product in each experiment are shown in Table 1.

**Table 1**

| | Cooling temperature [°C] | 3-Chloropyruvic acid [wt%] | Dichloropyruvic acid [wt%] | Trichloropyruvic acid [wt%] | Residual pyruvic acid [wt%] |
|---|---|---|---|---|---|
| Experiment 1 | -4.5 | 21.6 | 2.0 | 0.0 | 76.4 |
| Experiment 2 | 1.0 | 63.8 | 2.5 | 0.0 | 33.7 |
| Experiment 3 | 5.5 | 82.0 | 4.7 | 0.0 | 13.3 |
| Experiment 4 | 9.5 | 75.4 | 11.3 | 0.0 | 14.3 |
| Experiment 5 | 12.5 | 70.2 | 13.2 | 1.1 | 15.5 |

### Example 2

The 3-chloropyruvic acid obtained in Experiment 3 of Example 1 was purified using the fractional sublimation purification apparatus shown in Fig. 2. The internal container had an outer diameter of 70 mm, the external container had an inner diameter of 95 mm and both were made of general-purpose Pyrex glass (thickness: 3 mm). The length of the external container beneath the flange was 210 mm, and the gap between the bottom of the external container and the bottom of the internal container was 40 mm. The inside volume of the internal container was approximately 0.7 liters, and this was filled with ice water to cool the internal container to 0°C.

### Experiment 6

23.8 g of 3-chloropyruvic acid obtained in Experiment 3 were placed in the external container, both containers were assembled and a vacuum was drawn between both containers. The first sublimation purification step was performed for 1 hour setting the first heating temperature to 50°C. Following completion of this step, the container was removed from the constant-temperature bath, allowed to cool to room temperature, the vacuum was broken, and internal and external containers were separated, and the sublimed substance adhered to the outside of the internal container was removed. The amount of this sublimed substance (first fraction component) was 4.3 g. Analysis of the composition of this substance indicated that it consisted of 52.0 wt% of 3-chloropyruvic acid, 44.7 wt% of pyruvic acid and 3.3 wt% of dichloropyruvic acid.

After again assembling both containers and drawing a vacuum between them, the second sublimation purification step was performed for 1 hour setting the second heating temperature to 75°C. After breaking the vacuum and separating the internal container, the sublimed substance adhered to the outside of the internal container was recovered. The amount of this sublimed substance (second fraction component) was 14.1 g. When its composition was analyzed by NMR, it was found to consist of 90.8 wt% of 3-chloropyruvic acid, 5.2 wt% of pyruvic acid and 4.0 wt% of dichloropyruvic acid. In addition, the product yield [%] (= weight after purification ÷ weight before purification x 100) was 59.2%.

### Experiments 7 to 10

The purification procedure was performed under the same conditions as in Experiment 6 with the exception of setting the first and second heating temperatures to the values shown in Table 2. As a result, the amount of the first fraction component, amount of the second fraction component and compositions were as shown in Table 2, and the product purity and yield in Experiments 6 through 8 were better than those obtained in Experiments 9 and 10.

### Comparative Examples 1 and 2

The purification procedure was performed under the same conditions as in Experiment 6 with the exception of performing the sublimation purification step for 2 hours and in 1 step at the temperatures shown in Table 2. As a result, product (sublimed component) amounts and compositions were as shown in Table 2, and no improvements in purity were observed.

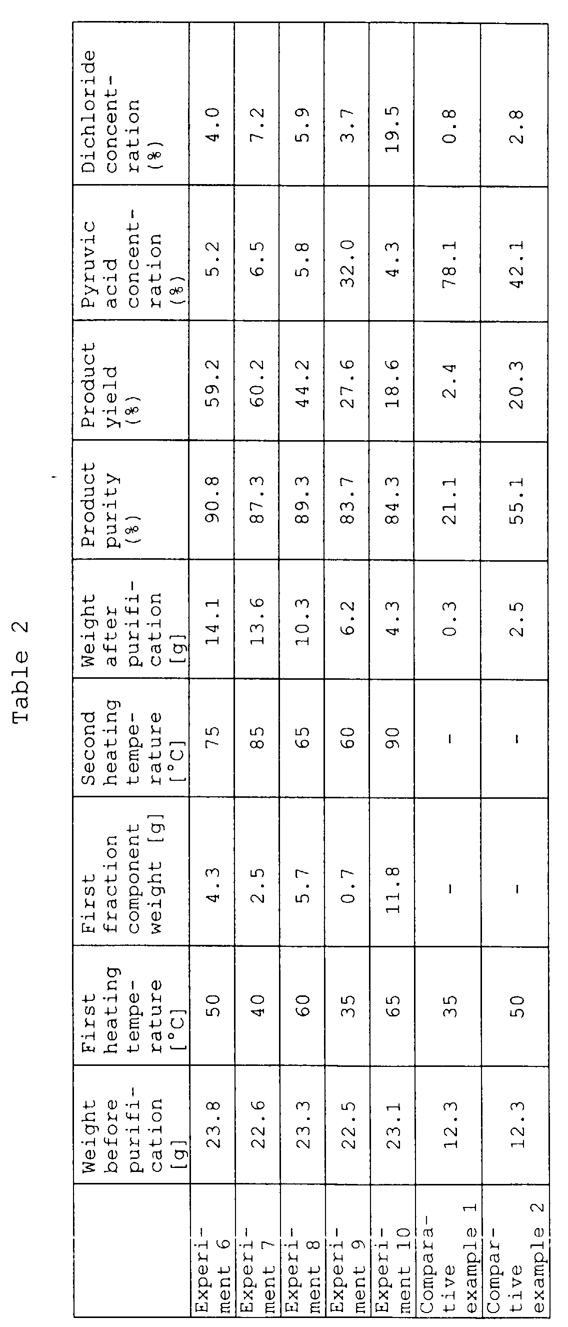

## Claims

1. A process for synthesizing 3-chloropyruvic acid by reacting pyruvic acid with sulfuryl chloride, comprising the steps of mixing said pyruvic acid and said sulfuryl chloride at a temperature lower than a reaction temperature; and, heating the mixture obtained in said preceding step to the reaction temperature to effect reaction between them.

2. The process for synthesizing 3-chloropyruvic acid according to Claim 1 wherein said temperature lower than a reaction temperature in said mixing step is 0 to 10°C.

3. The process for synthesizing 3-chloropyruvic acid according to Claim 1 wherein 3-chloropyruvic acid obtained in said reaction step is purified by temperature-controlled fractional sublimation.

4. The process for synthesizing 3-chloropyruvic acid according to Claim 3 wherein temperature control in said purification step is performed at a first heating temperature and a second heating temperature higher than said first heating temperature.

5. The process for synthesizing 3-chloropyruvic acid according to Claim 4 wherein said first heating temperature is 40 to 60°C, and said second heating temperature is 65 to 85°C.

6. The process for synthesizing 3-chloropyruvic acid according to Claim 4 wherein a component sublimed at said first heating temperature is removed, and a component sublimed at said second heating temperature is collected as purified 3-chloropyruvic acid.
